# EUROPEAN PATENT APPLICATION

(11) **EP 3 884 875 A1**
(43) Date of publication of application: **29.09.2021**
(21) Application number: 20165912.5
(22) Date of filing: 26.03.2020
(51) Int. Cl.: A61B 10/00, A61B 10/02

(54) **ASPIRATION DEVICE**

(71) Applicant: Universität Zürich, 8006 Zürich (CH)
(72) Inventor: Hecker, Andreas, 8700 Küsnacht (CH); Bouaicha, Samy, 8044 Zürich (CH)
(74) Representative: Latscha Schöllhorn Partner AG

(57) **Abstract**

The present invention relates to an aspiration device (1) for shoulder joint aspirations. The aspiration device (1) comprises a catheter unit (3) and a needle unit (2). The needle unit (2) is configured to be inserted into the catheter unit (3). The catheter unit (3) has a catheter sheath (15) made of a flexible plastics material. Further, the catheter sheath (15) comprises multiple lateral openings (13a, 13b) in its distal end region (E_{D}). The invention also relates to a catheter unit (3) for such an aspiration device (1).

## Description

### Technical Field

The present invention relates to an aspiration device according to the preamble of independent claim 1 and to a catheter unit for such a device.

### Background Art

In contrast to other puncturing operations, such as for example thoracentesis, arthrocentesis of the shoulder joint is not well explored yet. Especially regarding sensitivity no meaningful data is available up until now. However, it is presumed that the rate of dry aspirations is relatively high.

Currently, a radiologically controlled anterior approach in supine position appears to be the most effective way to perform shoulder aspirations in a high volume center. Other positions can lead to problems if the patient collapses and the adjustment of the x-ray beam is more time consuming. On the other hand, the supine position is not perfect to achieve the main goal of fluid aspiration if not much fluid is present in the joint because it is located in the posterior recess.

Thus, there is an existing need to approach the technical problems of shoulder joint aspirations and to propose possible approaches that might reduce the rate of dry aspirations and maximize the amount of the obtained fluid.

In WO 2015/033212 A1 an aspiration device is described, which includes an aspiration unit for extracting fluid from an aspiration site on a patient's body, as for example the pleural cavity in case of an aspiration of the lungs for thoracentesis. The aspiration unit comprises a cannula for piercing the aspiration site and a catheter connected to the cannula. The catheter is adapted to be inserted in the aspiration site to extract the fluid. A stabilizer is provided to affix the aspiration unit to the patient's body. The stabilizer includes a guide having a guiding slot for guiding the movement of the aspiration unit while piercing the aspiration site. A collection bag adapted to be coupled to the catheter is provided for collecting the fluid extracted by the aspiration unit. Further, a transfer tube adapted to couple the collection bag and the aspiration unit is provided to transfer the fluid from the aspiration unit to the collection bag.

Yet, the above solution does not address the problem of how such an aspiration device shall be designed specifically for arthrocentesis applications in order to ensure a satisfactory fluid yield.

It is therefore the object of the present invention to overcome the above-described shortcomings and to provide for an aspiration device which is particularly advantageous when used for carrying out joint aspirations such as shoulder joint aspirations.

### Disclosure of the Invention

According to the invention these needs are settled by an aspiration device as it is defined by the features of independent claim 1. Preferred embodiments are subject of the dependent claims.

In one aspect, the present invention is an aspiration device for joint fluid aspiration, in particular, shoulder joint aspiration. The aspiration device comprises a catheter unit and a needle unit. The needle unit is configured to be inserted into the catheter unit. The catheter unit has a catheter sheath made of a flexible plastics material advantageously made of plastic and the catheter sheath comprises multiple lateral openings at its distal end region.

Generally, the term "joint aspiration" refers to a procedure to remove fluid from the space in and/or around a joint of a human or animal being. Thereby, generally, a catheter and a needle may be used which can be coupled to a syringe..

The term "catheter" addresses a thin tube made from a medical grade material which may generally serve a broad range of functions. Catheters represent medical devices that can be inserted into a human or animal body to treat diseases or perform a surgical procedure. By modifying the material or adjusting the way catheters are manufactured, it is possible to tailor catheters for specific applications.

Here, the catheter comprises a typically thin, flexible tube respectively sheath and thus represents a so-called "soft" catheter.

The term "needle" addresses a medical tool which enters the human or animal skin/body and comprises a hollow tube with a sharp tip that contains a small opening at a pointed end. Needles are commonly used with syringes (a hand-operated device with a plunger), to inject substances into the body or to extract fluids respectively fluid samples from the body.

The term "distal" as used in connection with the invention and the disclosed embodiments thereof can relate to an orientation towards a body of a patient to be treated by the aspiration device. More specifically, the distal end region of the catheter sheath is a region in vicinity of a longitudinal end of the catheter sheath inserted into the joint of the patient while aspirating the joint. Contrary, the term "proximal" as used in connection with the invention and the disclosed embodiments thereof can relate to an orientation directed away from the body of the patient. As an example, in a conventional syringe the distal end usually is the tip of the needle and the proximal end is the end of the plunger where the thumb is to be laid.

The "lateral openings" of the catheter sheath are particularly designed for suctioning fluid such as a synovial fluid from human or animal joints and in particular from the shoulder joint.

By having the needle unit, the aspiration device according to the invention can efficiently puncture the skin and tissue of the patient in order introduce. Since the needle unit can be inserted into the catheter unit, the catheter unit and in particular its catheter sheath can be forwarded beyond the needle unit into the joint once the joint is made accessible by the needle unit. Then, the flexibility of the catheter sheath allows for conveniently accessing more or less the complete joint. Moreover, by comprising multiple openings, the fluid can be efficiently withdrawn and clogging of the catheter sheath can be prevented. Thus, by having the comparably rigid needle unit together with the flexible multi-holed catheter sheath, the aspiration device allows for efficiently introducing the catheter unit into the joint and at the same time for comparably completely aspirating fluid out of the joint.

Preferably, the catheter sheath of the catheter unit is equipped with a radiopaque element. The radiopaque element generally serves for indicating the positioning of the catheter to the practitioner and thus represents an assisting safety feature. Advantageously, the radiopaque element is in the form of a stripe, band or cable which may extend approximately over the entire length of the catheter sheath (i.e. in the longitudinal direction of the catheter sheath).

The term "radiopacity" generally defines opacity to the radio wave and X-ray portion of the electromagnetic spectrum or, in other words, the relative inability of those kinds of electromagnetic radiation to pass through a particular material. Materials that inhibit the passage of electromagnetic radiation are called radiopaque, while those that allow radiation to pass more freely are referred to as radiolucent.

Radiopaque volumes of material have white appearance on radiographs, compared with the relatively darker appearance of radiolucent volumes. For example, on typical radiographs, bones look white or light gray (radiopaque), whereas muscle and skin look black or dark gray, being mostly invisible (radiolucent).

In the present case, the radiopaque stripe may be made of barium sulfate, bismuth, tungsten or blends thereof. These materials have proven to be very efficient in use.

Preferably, the catheter sheath comprises six to fourteen openings, preferably ten openings, in the distal region. It has been found that with a number of openings within this range, relatively high volumes of synovial fluid may be aspirated efficiently and conveniently, in particular, from shoulder joints.

Preferably, the openings are arranged in two to six rows, preferably four rows, which are offset form one another in a circumferential direction of the catheter sheath. Advantageously, the offset of the rows is uniformly at an angle of between approximately 60° to 180°, preferably 90°. In this manner, the yield of synovial fluid aspiration may be further optimized.

Thereby, for example, two rows with three openings, respectively, are arranged opposite from one another and two rows with two openings, respectively, are arranged opposite from one another, wherein all four rows are offset from another by an angle of 90°. The openings of the rows with the two openings are preferably arranged (offset) in the spaces between the openings of the rows with three openings.

However, it is also possible that the offset of the rows is non-uniformly. Referring to the above example this means that e.g. the offset angle between the individual rows varies between approximately 10° and approximately 170°. It is also conceivable that the two rows with the three openings, respectively, are not arranged opposite from one another and/or the two rows with the two openings, respectively, are not arranged opposite from one another, wherein again the offset angle between the rows may vary between approximately 10° and approximately 170°. This however depends on the specific circumstances of the individual case, respectively.

Preferably, the openings comprise a diameter of between approximately 0.5 mm to approximately 2.0 mm, preferably between approximately 0.75 mm to approximately 1.75 mm. These dimensions have proven to be particularly suitable for shoulder joint aspirations.

Preferably, the distance between the openings in one row is between approximately 4 mm to approximately 6 mm, preferably approximately 5 mm. In this manner a particularly uniform suctioning of the synovial liquid may be achieved and the mechanical properties of the catheter are not relevant impaired.

Preferably, the openings are arranged at least in a distance of approximately 5 mm from the tip of the catheter sheath. Herewith, in can be ensured that a uniform suctioning is still possible if the opening in the tip of the catheter sheath is partially or fully clogged with e.g. synovial tissue.

Preferably, the diameter of the catheter sheath is between approximately 0.5 mm to approximately 2.0 mm, preferably approximately 0.8 mm to approximately 1.7 mm. Further preferred, the needle unit comprises a 14 gauge, a 16 gauge, a 18 gauge, a 20 gauge or a 22 gauge steel needle. The needle unit advantageously has a length between approximately 50 mm to approximately 150 mm, preferably between approximately 70 mm to approximately 133 mm. The catheter unit preferably comprises analogue gauge sizes and lengths. These dimensions of the catheter unit and the needle unit are particularly suitable for arthrocentesis applications respectively shoulder joint aspirations.

Preferably, the catheter sheath is made of latex, silicone rubber, nylon, polyurethane (PUR), polyethylene terephthalate (PET) or thermoplastic elastomer (TPE). With these materials relatively soft catheter sheaths may be provided which are generally less invasive/painful for the patient in operation and which allow an efficient complete fluid aspiration.

Preferably, the needle unit and/or the catheter unit comprise(s) a syringe connector at the proximal end. Like this, full operability of the inventive aspiration device may be provided.

In another aspect, the invention is a catheter unit for an aspiration device as described above or any of the preferred embodiments described above.

In yet another aspect, the invention is directed to a method of carrying out shoulder joint aspirations with an inventive aspiration device, the method comprising the following steps: (a) identifying the bony landmarks of the shoulder (especially of the coracoid process); (b) performing an anterior-posterior X-ray to receive a true anterior-posterior view (especially the coracoid process and the glenoid are identified on the X-ray); (c) inserting the needle unit with the catheter unit through the patient's skin (directly) lateral to the coracoid process; (d) advancing the needle unit aiming to the upper third of the glenoid (after passing the shoulder joint capsule a loss of resistance should be felt); (e) performing an X-ray control (the needle tip needs to be directly lateral to the glenoid in is upper third); (f) keeping the needle unit in place, while the catheter unit is advanced into the gleno-humeral articulation (no resistance should be felt by doing so, the catheter unit should be advanced as far as possible); (g) optionally, performing another X-ray which shows a radiopaque element/stripe of the catheter sheath (i.e. which represents the course of the catheter unit; however, this step can be skipped if the practitioner is sure about the intra-articular placement of the catheter unit); (h) removing the needle unit while the catheter unit is kept in place; (i) attaching a syringe to the catheter unit and (slowly) pulling the plunger; (j) keeping the catheter unit in this position as long joint fluid is aspirated (if no more fluid is obtained, the catheter unit is slowly pulled out of the joint while negative pressure is maintained by pulling the plunger - if joint fluid is again streaming into the syringe doing this maneuver, the catheter unit is kept in place until no more fluid is obtained and then again pulled back a little); (k) pulling the catheter unit out of the joint and the skin after aspiration and closing the puncture wound with a sterile dressing.

### Brief Description of the Drawings

The aspiration device according to the invention as well as the inventive method and the inventive system are described in more detail herein below by way of exemplary embodiments and with reference to the attached drawings, in which:
- Fig. 1: shows a side view of an inventive aspiration device with catheter unit and needle unit;
- Fig. 2: shows a side view of the needle unit for the inventive aspiration device; and
- Fig. 3: shows a side view of the catheter unit for the inventive catheter device.

### Description of Embodiments

In the following description certain terms are used for reasons of convenience and are not intended to limit the invention. The terms "right", "left", "up", "down", "under" and "above" refer to directions in the figures. The terminology comprises the explicitly mentioned terms as well as their derivations and terms with a similar meaning. Also, spatially relative terms, such as "beneath", "below", "lower", "above", "upper", "proximal", "distal", and the like, may be used to describe one element's or feature's relationship to another element or feature as illustrated in the figures. These spatially relative terms are intended to encompass different positions and orientations of the devices in use or operation in addition to the position and orientation shown in the figures. For example, if a device in the figures is turned over, elements described as "below" or "beneath" other elements or features would then be "above" or "over" the other elements or features. Thus, the exemplary term "below" can encompass both positions and orientations of above and below. The devices may be otherwise oriented (rotated 90 degrees or at other orientations), and the spatially relative descriptors used herein interpreted accordingly. Likewise, descriptions of movement along and around various axes include various special device positions and orientations.

To avoid repetition in the figures and the descriptions of the various aspects and illustrative embodiments, it should be understood that many features are common to many aspects and embodiments. Omission of an aspect from a description or figure does not imply that the aspect is missing from embodiments that incorporate that aspect. Instead, the aspect may have been omitted for clarity and to avoid prolix description. In this context, the following applies to the rest of this description: If, in order to clarify the drawings, a figure contains reference signs which are not explained in the directly associated part of the description, then it is referred to previous or following description sections. Further, for reason of lucidity, if in a drawing not all features of a part are provided with reference signs it is referred to other drawings showing the same part. Like numbers in two or more figures represent the same or similar elements.

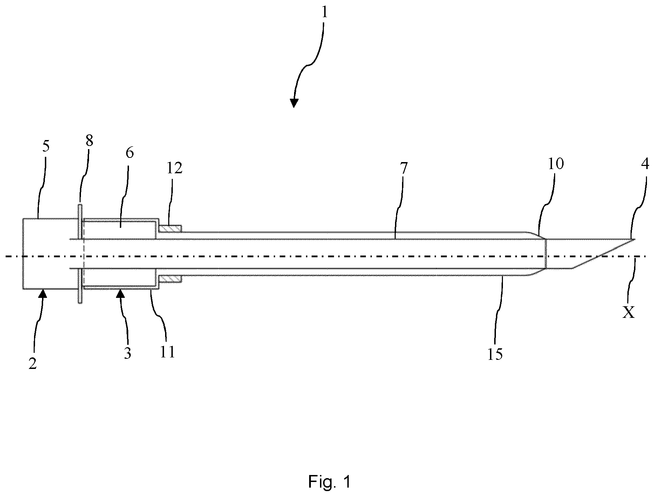

**Fig. 1** shows an inventive aspiration device 1 wherein the needle unit 2 has been inserted into the catheter unit 3 along the longitudinal axis X. At the distal end of the aspiration device 1, the needle tip 4 projects from the tapered catheter tip 10 and the catheter sheath 15 encloses the needle shaft 7. The needle unit 2 abuts with its flange portion 8 at the syringe connector 11 of the catheter unit 3, wherein the syringe connector 11 of the catheter unit 3 accommodates a corresponding coupling portion 6 of the needle unit 2. A holding portion 12 of the catheter unit 3 surrounds catheter sheath 15. At the proximal end of the aspiration device 1, the syringe connector 5 of the needle unit 2 is arranged. It is noted that it is generally also possible to inject substances through the needle unit 2 into a human or animal joint or body cavity. In connection with joint punctures, it is for example common practice to inject contrast agents into a human or animal joint for diagnostic reasons.

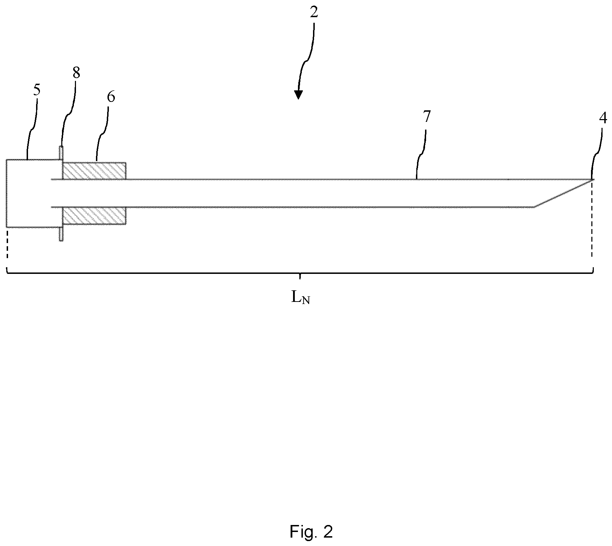

In **Fig. 2**, a needle unit 2 for the inventive aspiration device 1 is illustrated. The syringe connector 5 with flange portion 8 is followed by coupling portion 6 which surrounds a proximal end portion of the needle shaft 7. The proximal end of the needle shaft 7 projects into the syringe connector 5. The length L_{N} of the needle unit 2, measured from the needle tip 4 to the proximal end of the syringe connector 5 is between approximately 50 mm to approximately 150 mm, preferably between approximately 70 mm to approximately 133 mm. The needle unit may comprise a 14 gauge, a 16 gauge, an 18 gauge, a 20 gauge or a 22 gauge steel needle.

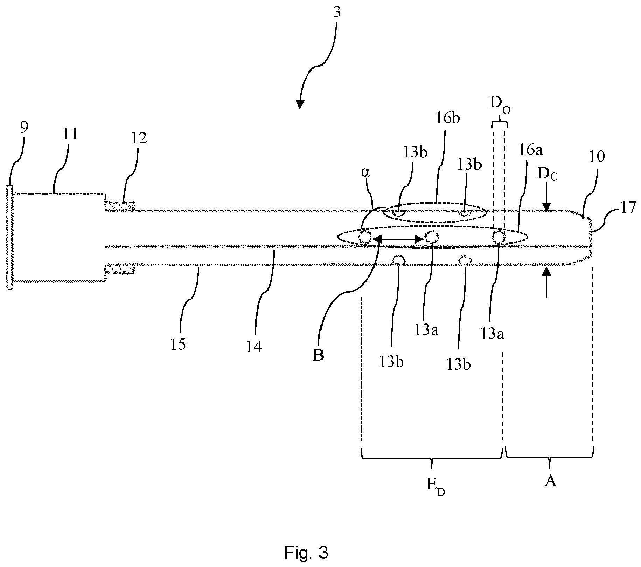

A catheter unit 3 for an inventive aspiration device 1 is shown in **Fig. 3****.** The syringe connector 11 with (proximal) flange portion 9 is followed by the catheter sheath 15 which is surrounded by holding portion 12. The catheter sheath 15 comprises tapered catheter tip 10 and has a diameter D_{C} between approximately 0.5 mm to approximately 2.0 mm, preferably approximately 0.8 mm to approximately 1.7 mm. In the distal end region E_{D} of catheter sheath 15 multiple lateral openings 13a and 13b are arranged. These lateral openings 13a and 13b serve for supporting the aspiration of synovial fluid in particular when the catheter tip opening 17 is clogged by synovial tissue.

The most distal openings 13a or 13b are arranged at a distance A of at least 5 mm from the tapered tip 10 of catheter sheath 15. In the present embodiment, the catheter sheath 15 comprises 10 openings 13a and 13b in the distal region E_{D}. The openings 13a and 13b are arranged in four rows 16a and 16b which are offset from one another in a circumferential direction of the catheter sheath 15. Here, the offset of the rows 16a and 16b is uniformly at an angle α of approximately 90°. Further, the two rows 16a with the three openings 13a are arranged directly opposite from one another and the two rows 16b with the two openings are arranged directly opposite from one another.

The rows 16a with the three openings 13a and the rows 16b with the two openings 13b are also offset from one another in the longitudinal direction of the catheter sheath 15 such that approximately in the middle between two openings 13a one opening 13b is arranged. The openings 13a and 13b comprise a diameter D_{O} of between approximately 0.5 mm to approximately 2.0 mm, preferably between approximately 0.75 mm to approximately 1.75 mm. The distance B between two openings 13a in one row 16a or between two openings 13b in one row 16b is between approximately 4 mm to approximately 6 mm, preferably approximately 5 mm.

The radiopaque element respectively stripe 14 is arranged in the catheter sheath 15, preferably within the wall of the catheter sheath 15. Advantageously, the radiopaque element respectively stripe extends over approximately the entire length of the catheter sheath 15.

This description and the accompanying drawings that illustrate aspects and embodiments of the present invention should not be taken as limiting-the claims defining the protected invention. In other words, while the invention has been illustrated and described in detail in the drawings and foregoing description, such illustration and description are to be considered illustrative or exemplary and not restrictive. Various mechanical, compositional, structural, electrical, and operational changes may be made without departing from the spirit and scope of this description and the claims. In some instances, well-known circuits, structures and techniques have not been shown in detail in order not to obscure the invention. Thus, it will be understood that changes and modifications may be made by those of ordinary skill within the scope and spirit of the following claims. In particular, the present invention covers further embodiments with any combination of features from different embodiments described above and below.

The disclosure also covers all further features shown in the figures individually although they may not have been described in the afore or following description. Also, single alternatives of the embodiments described in the figures and the description and single alternatives of features thereof can be disclaimed from the subject matter of the invention or from disclosed subject matter. The disclosure comprises subject matter consisting of the features defined in the claims or the exemplary embodiments as well as subject matter comprising said features.

Furthermore, in the claims the word "comprising" does not exclude other elements or steps, and the indefinite article "a" or "an" does not exclude a plurality. A single unit or step may fulfil the functions of several features recited in the claims. The mere fact that certain measures are recited in mutually different dependent claims does not indicate that a combination of these measures cannot be used to advantage. The terms "essentially", "about", "approximately" and the like in connection with an attribute or a value particularly also define exactly the attribute or exactly the value, respectively. The term "about" in the context of a given numerate value or range refers to a value or range that is, e.g., within 20%, within 10%, within 5%, or within 2% of the given value or range. Components described as coupled or connected may be electrically or mechanically directly coupled, or they may be indirectly coupled via one or more intermediate components. Any reference signs in the claims should not be construed as limiting the scope.

### List of reference numbers:

- 1: aspiration device
- 2: needle unit (cannulla)
- 3: catheter unit
- 4: needle tip
- 5: syringe connector (needle unit)
- 6: coupling portion (needle unit)
- 7: needle shaft
- 8: flange portion (needle unit)
- 9: flange portion (catheter unit)
- 10: tapered catheter tip
- 11: syringe connector (catheter unit)
- 12: holding portion
- 13a: openings (rows with three openings)
- 13b: openings (rows with two openings)
- 14: radiopaque element/stripe
- 15: catheter sheath
- 16a: rows with three openings
- 16b: rows with two openings
- 17: catheter tip opening
- A: distance opening from catheter tip
- B: distance between openings
- D_{C}: diameter catheter shaft
- D_{O}: diameter openings
- E_{D}: distal end region
- L_{N}: length needle unit
- X: longitudinal axis
- α: offset angle (in circumferential direction)

## Claims

1. Aspiration device (1) for joint aspirations comprising a catheter unit (3) and a needle unit (2) wherein the needle unit (2) is configured to be inserted into the catheter unit (3), wherein the catheter unit (3) has a catheter sheath (15) made of a flexible material and wherein the catheter sheath (15) comprises multiple lateral openings (13a, 13b) at its distal end region (E_{D}).

2. Aspiration device (1) according to claim 1, wherein the catheter sheath (15) of the catheter unit (3) is equipped with a radiopaque element (14).

3. Aspiration device (1) according to claim 1 or 2, wherein the catheter sheath (15) comprises six to fourteen openings (13a, 13b), preferably ten openings (13a, 13b), in the distal region (E_{D}).

4. Aspiration device (1) according to claim 3, wherein the openings (13a, 13b) are arranged in two to six rows (16a, 16b), preferably four rows (16a, 16b), which are offset from one another in a circumferential direction of the catheter sheath (15).

5. Aspiration device (1) according to claim 4, wherein the offset of the rows (16a, 16b) is uniformly at an angle α of between approximately 60° to 180°, preferably 90°.

6. Aspiration device (1) according to claim 4, wherein the offset of the rows (16a, 16b) is non-uniform.

7. Aspiration device (1) according to any one of claims 3 to 6, wherein the openings (16a, 16b) comprise a diameter (D_{O}) of between approximately 0.5 mm to approximately 2.0 mm, preferably between approximately 0.75 mm to approximately 1.75 mm.

8. Aspiration device (1) according to any one of claims 4 to 7, wherein the distance (B) between the openings (13a, 13b) in one row (16a, 16b) is between approximately 4 mm to approximately 6 mm, preferably approximately 5 mm.

9. Aspiration device (1) according to any one of claims 3 to 8, wherein the openings (13a, 13b) are arranged at least in a distance (A) of approximately 5 mm from the tip (10) of the catheter sheath (15).

10. Aspiration device (1) according to any one of the preceding claims, wherein the diameter (Dc) of the catheter sheath is between approximately 0.5 mm to approximately 2.0 mm, preferably approximately 0.8 mm to approximately 1.7 mm.

11. Aspiration device (1) according to any one of the preceding claims, wherein the needle unit comprises a 14 gauge, a 16 gauge, a 18 gauge, a 20 gauge or a 22 gauge steel needle.

12. Aspiration device (1) according to any one of the preceding claims, wherein the needle unit has a length (L_{N}) between approximately 50 mm to approximately 150 mm, preferably between approximately 70 mm to approximately 133 mm.

13. Aspiration device (1) according to any one of the preceding claims, wherein the catheter sheath (15) is made of latex, silicone rubber, nylon, polyurethane (PUR), polyethylene terephthalate (PET) or thermoplastic elastomer (TPE).

14. Aspiration device (1) according to any one of the preceding claims, wherein the needle unit (2) and/or the catheter unit (3) comprise(s) a syringe connector (5, 11) at a proximal end.

15. Method of carrying out a shoulder joint aspiration with an aspiration device according to claim 1, the method comprising the steps:
(a) identifying bony landmarks of a shoulder of a patient;
(b) performing an X-ray, preferably an anterior-posterior X-ray, to receive a true anterior-posterior view;
(c) inserting a needle unit (2) of the aspiration device with the catheter unit (3) of the aspiration device through the patient's skin lateral to a coracoid process of the shoulder;
(d) advancing the needle unit (2), preferably aiming to the upper third of the glenoid of the shoulder;
(e) preferably performing an X-ray control of a position of the needle unit;
(f) advancing the catheter unit (3) into the shoulder joint while the needle unit (2) is kept in place;
(g) optionally, performing another X-ray which shows a radiopaque element/stripe (14) of the catheter sheath (15);
(h) removing the needle unit (2) while the catheter unit (3) is kept in place;
(i) attaching a withdrawal unit such as a syringe to the catheter unit (3) and aspirating joint fluid, for example by pulling a plunger of the syringe;
(j) keeping the catheter unit (3) in this position as long joint fluid is aspirated;
(k) pulling the catheter unit (3) out of the joint and the skin after aspiration; and
(h) preferably closing the puncture wound with a sterile dressing.
